(19) Europäisches Patentamt / European Patent Office / Office européen des brevets

(11) **EP 2 628 483 B1**

(12) **EUROPEAN PATENT SPECIFICATION**

(45) Date of publication and mention
of the grant of the patent:
**07.12.2016 Bulletin 2016/49**

(21) Application number: **11832460.7**

(22) Date of filing: **05.10.2011**

(51) Int Cl.:
*A61K 33/44* (2006.01)          *A61P 1/16* (2006.01)
*A61P 13/12* (2006.01)          *A61P 39/02* (2006.01)
*B01J 20/20* (2006.01)          *B01J 20/30* (2006.01)
*C01B 31/10* (2006.01)          *A61K 9/14* (2006.01)
*A61L 15/18* (2006.01)          *B01J 20/28* (2006.01)

(86) International application number:
**PCT/JP2011/072960**

(87) International publication number:
**WO 2012/050025 (19.04.2012 Gazette 2012/16)**

(54) **MEDICAL ADSORBENT AND METHOD FOR PRODUCING SAME**

MEDIZINISCHES ADSORBENS UND VERFAHREN ZU SEINER HERSTELLUNG

MÉDICAMENT ABSORBANT ET SON PROCÉDÉ DE FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.09.2011  JP 2011200213
12.10.2010  JP 2010229408**

(43) Date of publication of application:
**21.08.2013  Bulletin 2013/34**

(73) Proprietor: **Futamura Kagaku Kabushiki Kaisha
Nagoya-shi, Aichi 450-0002 (JP)**

(72) Inventors:
• **KUROKAWA, Hiroyuki
Minokamo-shi
Gifu 505-0024 (JP)**
• **HIBI, Keita
Minokamo-shi
Gifu 505-0024 (JP)**

• **KOUSAKA, Tsutomu
Nagoya-shi
Aichi 450-0002 (JP)**
• **SUZUKI, Keisuke
Nagoya-shi
Aichi 450-0002 (JP)**

(74) Representative: **TBK
Bavariaring 4-6
80336 München (DE)**

(56) References cited:
EP-B1- 1 440 692          WO-A1-92/06919
WO-A1-2005/094844      JP-A- 1 246 117
JP-A- 2 001 282          JP-A- 4 231 314
JP-A- 50 148 627          JP-A- 54 028 291
JP-A- 54 089 010          JP-A- 56 005 313
JP-A- H06 501 910          JP-A- 2006 111 604
JP-A- 2006 131 461

**Description**

Technical field

The present invention relates to a medical adsorbent for oral administration, comprising activated carbon obtained using refined cellulose or regenerated cellulose as a starting material, as well as a method for producing it, and particularly it relates to a medical adsorbent for oral administration, comprising cellulose-derived activated carbon with excellent selective adsorption performance for toxic substances and excellent adsorption performance, as well as to a method for producing it.

Background Art

Patients with kidney disease or liver disease accumulate toxic substances in the blood, which can cause encephalopathies such as uremia and impaired consciousness. The number of such patients is increasing year by year. Hemodialyzing artificial kidneys and the like are used for treatment of such patients, to remove the toxic substances out of the body. However, such artificial kidneys are problematic in that they must be handled by a professional for safety management, and create a physical, psychological and economical burden on patients during removal of blood out of the body, and they are therefore less than satisfactory.

Alternative methods to artificial organs have been developed, such as orally administered adsorbents that are ingested orally and adsorb toxic substances in the body, and are excreted out of the body (see PTL 1 and PTL 2). Such adsorbents, however, utilize the adsorption performance of the activated carbon, and their adsorptive capacity for toxins to be removed, and their selective adsorption for toxins over essential substances, has not been sufficient. Activated carbon generally has high hydrophobicity, and therefore presents a problem in that it is not suitable for adsorption of causative substances of uremia, and their precursors, which are typically ionic organic compounds such as arginine and creatinine.

In order to circumvent the problems of activated carbon adsorbents, the use of xylogen (xylem), petroleum-based or coal-based pitches as starting substances to form spherical resin compounds, and the preparation of agents for anti-nephrotic syndrome comprising activated carbon, using these as starting materials, has been reported (see PTL 3, for example). The aforementioned activated carbon is prepared using petroleum-based hydrocarbon (pitch) or the like as the starting substance, to a relatively uniform particle size, and is carbonized and activated. In addition, there has also been reported an adsorbent for oral administration that has the particle size of the activated carbon itself made relatively uniform and has the pore volume distribution of the activated carbon modified (see PTL 4). Thus, it has been a goal to achieve a relatively uniform particle size for activated carbon for medicinal use and improve the poor intestinal flow property, while simultaneously improving the adsorption performance of the activated carbon by adjustment of the pores. It is therefore ingested by many mild chronic renal insufficiency patients.

Activated carbon for medicinal use must be able to rapidly and efficiently adsorb causative substances of uremia, and their precursors. With existing activated carbon for medicinal use, however, it has been difficult to reduce the particle size while maintaining spherical shapes. In addition, adjustment of the pores in conventional activated carbon for medicinal use has not been satisfactory and the adsorption performance is not always sufficient, and therefore the daily dosage must be increased. Especially given the fact that chronic renal failure patients are restricted in their water consumption, the reduced water levels make swallowing a major source of distress for patients.

Furthermore, the gastrointestinal tract including the stomach and small intestine is an environment containing numerous substances that include compounds that are indispensable for physiological function, such as saccharides and proteins, and enzymes secreted by the intestinal wall. Consequently, there has been a demand for activated carbon for medicinal use having selective adsorption performance that prevents adsorption of compounds such as trypsin that are enzymes that are essential for physiological function, while allowing adsorption of arginine, creatinine and the like, which are considered to be causative substances of uremia.

The aforementioned adsorbents for oral administration employ petroleum pitch and thermosetting resins such as phenol resins as the starting materials. Because they depend on petroleum-derived starting materials, they are not at all preferable from a carbon neutral standpoint. The production energy cost of the starting materials is also very high, and therefore a demand exists for activated carbon products for medicinal use that are adsorbents for oral administration derived from biomass.

EP 1 440 692 B1 describes a medical adsorbent which comprises activated carbon obtained by carbonizing a spherical phenol resin in a nitrogen atmosphere at a temperature of 400-1000 °C, activating the carbonized spherical phenol resin at a temperature of 800-1000 °C, washing it with dilute hydrochloric acid, heat treating it at a temperature of 150-1000 °C in a mixed gas comprising oxygen and nitrogen and then sorting it; the activated carbon has an area to weight ratio of 500-2000 $m^2$/g, a pore volume of 0.2-1.0 mL/g and a packing density of 0.5-0.75 g/mL.

Citation List

Patent Literature

PTL 1: Japanese Patent Publication No. 3835698 (JP 3835698 B)
PTL 2: Japanese Unexamined Patent Publication No. 2008-303193 (JP 2008-303193 A)
PTL 3: Japanese Unexamined Patent Publication HEI No. 6-135841 (JP 6-135841 A)
PTL 4: Japanese Unexamined Patent Publication No. 2002-308785 (JP 2002-308785 A)

SUMMARY OF INVENTION

TECHNICAL PROBLEM

The present invention has been accomplished in light of this situation, and it provides a medical adsorbent for oral administration that has low dosage and excellent adsorptive capacity and selective adsorption for toxins to be removed, and is also economical and environmentally friendly.

SOLUTION TO PROBLEM

Specifically, the invention according to claim 1 relates to a medical adsorbent that comprises granular activated carbon that is activated carbon obtained by carbonization and activation of refined cellulose or regenerated cellulose, and that has a mean pore diameter of 1.5 to 2.2 nm, a BET specific surface area of 700 to 3000 $m^2$/g, a mean particle size of 100 to 1100 $\mu$m, a surface oxide content of 0.05 meq/g or greater, and a packing density of 0.4 to 0.8 g/mL.
The invention of claim 2 relates to a medical adsorbent according to claim 1 wherein the granular activated carbon is a therapeutic or prophylactic agent for kidney disease or liver disease, for oral administration.
The invention of claim 3 relates to a method for producing a medical adsorbent whereby in production of granular activated carbon according to claim 1, refined cellulose or regenerated cellulose is carbonized at 300°C to 700°C under a nitrogen atmosphere, and then subjected to steam-activation at 750°C to 1000°C, acid cleaning and heat treatment at 500°C to 800°C.
The invention of claim 4 relates to a method for producing a medical adsorbent whereby in production of granular activated carbon according to claim 1, refined cellulose or regenerated cellulose is impregnated into ammonium phosphate or a metal phosphate, carbonized at 300°C to 700°C under a nitrogen atmosphere, and then subjected to steam-activation at 750°C to 1000°C, acid cleaning and heat treatment at 500°C to 800°C.

ADVANTAGOUS EFFECTS OF INVENTION

Since the medical adsorbent according to the invention of claim 1 comprises granular activated carbon that is activated carbon obtained by carbonization and activation of refined cellulose or regenerated cellulose, and that has a mean pore diameter of 1.5 to 2.2 nm, a BET specific surface area of 700 to 3000 $m^2$/g, a mean particle size of 100 to 1100 $\mu$m, a surface oxide content of 0.05 meq/g or greater, and a packing density of 0.4 to 0.8 g/mL, it can serve as a medical adsorbent for oral administration that has low dosage and excellent adsorptive capacity and selective adsorption for toxins to be removed, and is also economical and environmentally friendly.
Since the medical adsorbent according to the invention of claim 2 employs the granular activated carbon according to the invention of according to claim 1 as a therapeutic or prophylactic agent for kidney disease or liver disease, for oral administration, it has a high effect for selective adsorption of causative substances of kidney disease or liver disease, and is promising as a therapeutic or prophylactic agent.
The method for producing a medical adsorbent according to the invention of claim 3 is a method whereby, in production of granular activated carbon according to claim 1, refined cellulose or regenerated cellulose is carbonized at 300°C to 700°C under a nitrogen atmosphere, and then subjected to steam-activation at 750°C to 1000°C, acid cleaning and heat treatment at 500°C to 800°C, and consequently it successfully establishes a method for producing a medical adsorbent that uses biomass-derived starting materials.
The method for producing a medical adsorbent according to the invention of claim 4 is a method whereby, in production of granular activated carbon according to claim 1, refined cellulose or regenerated cellulose is impregnated into ammonium phosphate or a metal phosphate, carbonized at 300°C to 700°C under a nitrogen atmosphere, and then subjected to steam-activation at 750°C to 1000°C, acid cleaning and heat treatment at 500°C to 800°C, and consequently it successfully establishes a method for producing a medical adsorbent that uses biomass-derived starting materials while also facilitating control of the physical properties of the granular activated carbon product.

DESCRIPTION OF EMBODIMENTS

The medical adsorbent of the invention is granular activated carbon obtained using refined cellulose or regenerated cellulose as the starting material, the cellulose starting material being carbonized and activated to develop pores. A refined cellulose starting material is cellulose obtained with increased purity by dissolving natural pulp with an acid or alkali and washing it. Regenerated cellulose is high-purity cellulose prepared from pulp by a conventionally known method such as the viscose method or copper-ammonia method.

Alternatively, it is cellulose prepared after dissolving pulp using an ionic liquid such as NMMO (N-methylmorpholine oxide) or BMIMCL (1-butyl-3-methylimidazolium chloride). For adjustment of the cellulose solution viscosity and the pore distribution of the cellulose aggregates, starch that is soluble in the cellulose starting material or water-insoluble may be added at up to 20 mass% (wt%). In order to further increase the strength of the activated carbon, cellulose fiber or inorganic fiber such as silica may be added as a filler, at up to 20 mass% (wt%).

The form of the refined cellulose and regenerated cellulose is preferably granular, considering it is to be ingested as a medical adsorbent. Especially in consideration of the flow property in the intestinal tract, the preferred form of activated carbon for medical use is spherical. Refined cellulose or regenerated cellulose can be obtained by solidification in water or a strong acid. Simple spherical cellulose particles are formed by dropping a prescribed concentration of the viscose solution in a solidifying solution of water or a strong acid, or by stirring and dispersion in a solidifying solution by a known method. The mean particle size of spherical cellulose particles is adjusted as desired by the concentration and viscosity of the viscose solution, the orifice diameter of the liquid discharge nozzle during solidification, and the rotational speed of the solidifying solution. The cellulose solution discharge apparatus is adjusted so that activated carbon with a final mean particle size of 100 to 1100 $\mu$m is obtained. The particle size is 150 to 2000 $\mu$m at the stage of dried granular cellulose, prior to carbonization.

A commonly considered use of cellulose particles is in cosmetic powders or pharmaceutical excipients. Because cellulose particles must have softness and self-disintegrating properties, they are not expected to exhibit any particular degree of hardness. Also, microcrystalline cellulose fine particles are used as molding accelerators for sphericalization of pharmaceuticals, and are formulated together with drugs to serve as nuclei in the drugs. With microcrystalline cellulose, however, even if it is possible to prepare hard spherical cellulose particles with a constant particle size, the hardness is not maintained in the body.

On the other hand, cellulose is a naturally derived component, and has the advantage of easier starting material procurement and starting material preparation. Also, a shorter time is required for activation compared to activated carbon of phenol-based resins. The present inventors have shown that by controlling the concentration when dissolving cellulose, adjusting the molecular polymerization degree of viscose, or mixing and impregnating a noncombustible component in order to increase the hardness, it is possible to modify the particle size and hardness within wide ranges. By further subjecting the obtained cellulose granules to carbonization and activation, a medical adsorbent of granular activated carbon with the desired hardness was successfully obtained even when using cellulose starting materials that have presented difficulties in the prior art.

The granular activated carbon as the main component of the medical adsorbent will now be described, starting from the method of production. First, as specified by the invention of claim 3, granular cellulose comprising the aforementioned refined cellulose or regenerated cellulose is set in a firing furnace such as a cylindrical retort electric furnace and carbonized in the furnace at a temperature between 300 and 700°C, under a nitrogen atmosphere, to form granular carbonized cellulose.

Alternatively, as specified by the invention of claim 4, the granular cellulose comprising refined cellulose or regenerated cellulose is impregnated into a solution of ammonium phosphate or a metal phosphate such as sodium phosphate or potassium phosphate. The granular cellulose containing the phosphate is set in a firing furnace such as a cylindrical retort electric furnace and carbonized in the furnace at a temperature between 300 and 700°C, under a nitrogen atmosphere, to form granular carbonized cellulose. The impregnation into the phosphate solution is carried out for flame retardance of the granular cellulose.

The granular carbonized cellulose obtained by either process described above is subjected to steam-activation at between 750 and 1000°C, preferably between 800 and 1000°C, and even more preferably between 850 and 950°C. The activation time will depend on the production scale and equipment, but may be between 0.5 and 50 hours. After cooling under a nitrogen atmosphere, the activated granular carbonized cellulose is subjected to acid cleaning with dilute hydrochloric acid or the like. Following acid cleaning, it is rinsed with water to remove the impurities such as ash. After acid cleaning, the activated granular carbonized cellulose is subjected to heat treatment for between 15 minutes and 2 hours in a gaseous mixture containing oxygen and nitrogen, to remove the residual hydrochloric acid content. The surface oxide content of the activated carbon is adjusted through each of the treatments. During the heat treatment, the oxygen concentration is controlled to be no greater than between 0.1 and 5 vol%. After acid cleaning, the activated granular carbonized cellulose is heated at between 500 and 800°C.

In either production method, the granular activated carbon that has completed the final heating is sifted with a sieve or the like for particle size adjustment and separation of the granular activated carbon. Granular activated carbon as a medical adsorbent according to the invention is thus obtained. The sifting removes activated carbon with large particle sizes, that has a slow adsorption rate and cannot sufficiently exhibit adsorptive power.

The granular activated carbon obtained by the production method described above must adsorb causative substances of impaired liver function or impaired renal function mentioned in the examples described below, while having absolutely minimal adsorption of enzymes necessary for the body, i.e. while exhibiting improved selective adsorption performance, and it must also exhibit sufficient adsorption performance at relatively low dosages. In order for the properties to be within a balanced range, the medical adsorbent of the invention is defined by the parameters of [1] mean pore diameter, [2] BET specific surface area, [3] mean particle size, [4] surface oxide content, and [5] packing density, as according to the invention of claim 1. Preferred ranges for the values of each parameter will also become apparent by the examples described below. The methods and conditions for measuring the physical properties of the activated carbon are described in detail in the examples.

First, the [1] mean pore diameter is specified to be between 1.5 and 2.2 nm. The mean pore diameter is preferably not less than 1.5 nm because the adsorption performance for toxic substances will be reduced. Conversely, the mean pore diameter is preferably not greater than 2.2 nm because more pores will be present that will adsorb high molecular compounds such as enzymes and polysaccharides that are necessary for the body. Therefore, the mean pore diameter is preferably within the aforementioned range, and more preferably between 1.6 and 2.0 nm.

The [2] BET specific surface area is specified to be between 700 and 3000 $m^2/g$. The BET specific surface area is preferably not less than 700 $m^2/g$ because the adsorption performance for toxic substances will be reduced. The BET specific surface area is also preferably not greater than 3000 $m^2/g$ because the packing density will be lowered and the pore volume increased, tending to impair the strength of the granular activated carbon itself. The BET specific surface area is therefore preferably within the aforementioned range, more preferably between 900 and 2400 $m^2/g$, and even more preferably between 1000 and 2000 $m^2/g$.

The [3] mean particle size is specified to be between 100 and 1100 $\mu$m. The mean particle size is preferably not smaller than 100 $\mu$m because useful substances such as digestive enzymes will tend to be adsorbed, which is undesirable from the viewpoint of selective adsorption. In addition, a mean particle size of less than 100 $\mu$m, such as 20 $\mu$m, is theoretically possible but difficult to produce in practice. If the mean particle size is larger than 1100 $\mu$m, the particles will become too large, reducing the relative surface area, and the adsorption rate will be lower as a result. The mean particle size is preferably within the range specified above, more preferably between 100 and 1000 $\mu$m, and even more preferably between 300 and 1000 $\mu$m. The term "mean particle size" as used herein refers to the particle size with an integral value of 50% in the particle size distribution determined by laser diffraction/scattering using a laser light scattering particle size distribution analyzer mentioned in the examples below.

The [4] surface oxide content is specified to be at least 0.05 meq/g. An increased surface oxide content on the granular activated carbon surface causes an increase in ionic functional groups on the activated carbon surface. In order to improve the adsorption performance of the ionic organic compound, therefore, the surface oxide content should be at least 0.05 meq/g, and even more preferably at least 0.10 meq/g. A surface oxide content of less than 0.05 meq/g cannot be considered suitable since the adsorption property will be inferior.

The [5] packing density is specified to be between 0.4 and 0.8 g/mL. If the packing density is less than 0.4 g/mL, the dosage will increase and swallowing during oral administration will become difficult. A packing density exceeding 0.8 g/mL is unsuitable because it will result in a poor balance with the desired selective adsorption. Therefore, the packing density is preferably within the range specified above, and preferably between 0.5 and 0.7 g/mL.

Granular activated carbon having the physical properties described above is an agent intended for oral administration, as specified by the invention of claim 2, and can serve as a therapeutic or prophylactic agent of kidney disease or liver disease. As mentioned above, if causative substances of diseases and chronic symptoms are adsorbed and retained in pores developed on the surface of granular activated carbon, and are excreted out of the body, then it is possible to prevent worsening of symptoms and improve pathology. In addition, prior internal use of granular activated carbon according to the invention for actual or possible congenital or acquired metabolic disorders can lower the concentration of causative substances of diseases and chronic symptoms in the body. Ingestion may also be considered for prevention to avoid worsening of symptoms.

Examples of kidney diseases include chronic renal failure, acute renal failure, chronic pyelonephritis, acute pyelonephritis, chronic nephritis, acute nephritis syndrome, acute advanced nephritis syndrome, chronic nephritis syndrome, nephrotic syndrome, renal sclerosis, interstitial nephritis, nephric tubular syndrome, lipoid nephrosis, diabetic nephropathy, renovascular hypertension, hypertension syndrome, and secondary kidney disease complications of the aforementioned conditions, as well as mild renal insufficiency before dialysis. Examples of liver diseases include fulminant hepatitis, chronic hepatitis, viral hepatitis, alcoholic hepatitis, hepatic fibrosis, hepatic cirrhosis, liver cancer, autoimmune hepatitis, drug allergenic hepatopathy, primary biliary liver cirrhosis, thrill, encephalopathy, metabolic disorders and dysfunction.

The dosage for using granular activated carbon according to the invention as an oral medical adsorbent cannot be specified for all cases as it will be affected by age, gender, physical constitution and symptoms. For most human patients, however, administration of granular activated carbon 2 to 4 times at 1 to 20 g per day based on body weight may be assumed. The oral medical adsorbent of granular activated carbon is administered in a type and dosage form such as powder, granules, tablets, sugar-coated tablets, capsules, or a suspending agent, stick agent, divided powder package or emulsion.

Examples

Methods for measuring parameters

The present inventors measured the physical properties of mean particle size ($\mu$m), BET specific surface area ($m^2$/g), pore volume (mL/g), mean pore diameter (nm), packing density (g/mL) and surface oxide content (meq/g), for the granular activated carbons of the examples and comparative examples described below. Also simultaneously evaluated were the adsorption performance for the toxic substances creatinine and arginine (toxic causative substances), and the adsorption performance for the essential substance trypsin. In addition, the iodine adsorptive power (mg/g) was measured to evaluate the general adsorption performance of the activated carbon.

The mean particle size ($\mu$m) was measured using a laser light scattering particle size distribution analyzer (SALD3000S) by Shimadzu Corp., and the particle size was recorded as 50% of the integrated value in the particle size distribution determined by laser diffraction/scattering.

The BET specific surface area ($m^2$/g) was determined by the BET method, measuring the nitrogen adsorption isotherm at 77K using a BELSORP mini by Bel Japan, Inc.

The following two methods were used for the pore volume (mL/g).

The $N_2$ pore volume $V_{mi}$ was determined by applying the Gurvitsch law, using a BELSORPmini by Bel Japan, Inc., based on the nitrogen adsorption in terms of liquid nitrogen at a relative pressure of 0.990. The same method was used for a pore diameter range of 0.6 to 100 nm.

The mercury pore volume $V_{me}$ was determined using an AUTOPORE 9500 by Shimadzu Corp., with the contact angle set to 130° and the surface tension set to 484 dyne/cm (484 mN/m), measuring the pore volume by the mercury porosimetry for pore diameters of 7.5 to 15,000 nm.

The mean pore diameter Dp (nm) was determined by the following formula (i), assuming circular cylindrical pore shapes. In the formula, $V_{mi}$ is the $N_2$ pore volume, and Sa is the BET specific surface area.

Formula 1

$$D P (nm) = \left[ \frac{(4 \times V_{mi})}{Sa} \right] \times 1000 \quad \cdots (i)$$

The packing density (g/mL) was measured according to JIS K 1474(2007).

The surface oxide content (meq/g) was the amount of sodium hydroxide determined by applying the Boehm method, shaking the granular activated carbon in a 0.05N sodium hydroxide water-soluble solution and filtering it, and conducting neutralization titration with the filtrate using 0.05N hydrochloric acid.

The iodine adsorptive power (mg/g) was measured according to JIS K 1474(2007).

For examples of substances to be adsorbed, creatinine and arginine were used as toxic substances and trypsin was used as an essential substance, and the adsorption performance by the granular activated carbon of each test example was evaluated. First, each substance to be adsorbed was dissolved in phosphate buffer at pH 7.4, to prepare a standard solution with the concentration of 0.1 g/L for each substance to be adsorbed.

The granular activated carbon of the examples and comparative examples were each added at 2.5 g to 50 mL of creatinine standard solution, and contact stirred for 3 hours at a temperature of 37°C.

The granular activated carbon of the examples and comparative examples were each added at 0.5 g to 50 mL of arginine standard solution, and contact stirred for 3 hours at a temperature of 37°C.

The granular activated carbons of the examples and comparative examples were each added at 0.125 g to 50 mL of trypsin standard solution, and contact stirred for 3 hours at a temperature of 21°C.

The filtrate obtained by subsequent filtration was used for measurement of the TOC concentration (mg/L) in each filtrate using a total-organic carbon meter (TOC5000A by Shimadzu Corp.), and the mass of the substance to be adsorbed in each filtrate was calculated. The adsorption rate (%) for each substance to be adsorbed was determined by the following formula (ii) .
Formula 2

$$Adsorption\ rate(\%) = \left\{ \frac{standard\ solution\ concentration - filtrate\ concentration}{standard\ solution\ concentration} \right\} \times 100 \ ... \ (ii)$$

Production of granular activated carbon for examples and comparative examples

Example 1

A 2 kg portion of dissolved pulp LNDP (product of Nippon Paper Chemicals Co., Ltd.) comprising 90 mass% (wt%) α-cellulose per unit weight was dipped with a sodium hydroxide solution (18.5% concentration) at 55°C for 15 minutes, and it was pressed to remove the excess sodium hydroxide content to prepare alkali cellulose (AC) with a cellulose concentration of 33.5 mass% (wt%). The alkali cellulose was aged at 40°C for 7 hours, and 5 kg of the alkali cellulose was reacted with 436 mL of carbon disulfide of ≥97% purity for 70 minutes, to obtain cellulose xanthate having a viscosity of 0.055 Pa.s (55 cP) at 40°C.

Upon completion of the reaction, approximately 13 L of a dilute sodium hydroxide solution was added to the cellulose xanthate, and the mixture was stirred for 100 minutes to obtain viscose. It was also subjected to defoaming, maturation and filtration steps to prepare viscose with a cellulose concentration of 9.0%. The prepared viscose was diluted to a viscose concentration of 80% with distilled water, and was dropped into a gently stirred large excess of a dilute sulfuric acid bath (coagulating bath) at 40°C from a nozzle with an inner diameter of about 0.9 mm (18 gauge), regenerating the cellulose, to obtain spherical cellulose (regenerated cellulose). Immersion of the spherical cellulose in the dilute sulfuric acid bath was for 30 minutes. After rinsing the spherical cellulose with an excess of water to remove the dilute sulfuric acid, it was dipped for 1 hour in a dilute sodium hydroxide water-soluble solution at 40°C. After rinsing again in an excess of water to remove the sodium hydroxide content in the spherical granules, drying was performed at 105°C to obtain spherical cellulose.

To 250 g of the spherical cellulose obtained by this preparation there was added 500 mL of an ammonium phosphate water-soluble solution (5% concentration), and the mixture was allowed to stand for 12 hours. The water was then drained, and the mixture was dried at 120°C for 3 hours with a dryer. The total amount of the ammonium phosphate-treated spherical cellulose was placed in a cylindrical retort electric furnace, nitrogen was charged in, and then the temperature was raised to 600°C at 100°C/hr and held at that temperature for 1 hour for carbonization. The carbonized product was then heated to 900°C, water vapor was added and the mixture was held at that temperature for 1 hour for activation (activation temperature: 900°C, activation time: 1 hour). The activated carbon was washed with dilute hydrochloric acid, nitrogen was charged in and heat treatment was performed in a cylindrical retort electric furnace at 600°C for 1 hour to obtain granular activated carbon for Example 1 (yield: 19.6%).

Example 2

Granular activated carbon for Example 2 was obtained in the same manner as Example 1, except that the activation temperature for Example 1 was changed to 750°C and the activation time was 2 hours (yield: 30.1%).

Example 3

Granular activated carbon for Example 3 was obtained in the same manner as Example 1, except that the activation temperature for Example 1 was changed to 750°C and the activation time was 8 hours (yield: 22.6%).

Example 4

During preparation of spherical cellulose for Example 4, viscose with a cellulose concentration of 9.0% was diluted to a viscose concentration of 50% with distilled water, and was dropped into a gently stirred large excess of a dilute sulfuric acid bath (coagulating bath) at 40°C from a nozzle with an inner diameter of about 0.9 mm (18 gauge), regenerating the cellulose, to obtain spherical cellulose (regenerated cellulose).

To 250 g of the spherical cellulose obtained by this preparation there was added 500 mL of an ammonium phosphate water-soluble solution (5% concentration), and the mixture was allowed to stand for 12 hours. The water was then drained, and the mixture was dried at 120°C for 3 hours with a dryer. The total amount of the ammonium phosphate-treated spherical cellulose was placed in a cylindrical retort electric furnace, nitrogen was charged in, and then the temperature was raised to 600°C at 100°C/hr and held at that temperature for 1 hour for carbonization. The carbonized product was then heated to 900°C, water vapor was added and the mixture was held at that temperature for 45 minutes for activation (activation temperature: 900°C, activation time: 0.75 hour). The activated carbon was washed with dilute hydrochloric acid, nitrogen was charged in and heat treatment was performed in a cylindrical retort electric furnace at 600°C for 1 hour to obtain granular activated carbon for Example 4 (yield: 14.4%).

Example 5

During preparation of spherical cellulose for Example 5, viscose with a cellulose concentration of 9.0% was diluted to a viscose concentration of 50% with distilled water, and was dropped into a gently stirred large excess of a dilute sulfuric acid bath (coagulating bath) at 40°C from a nozzle with an inner diameter of about 0.7 mm (19 gauge), regenerating the cellulose, to obtain spherical cellulose (regenerated cellulose).
The ammonium phosphate treatment, heating, steam-activation, dilute hydrochloric acid washing and heating treatments were all carried out according to Example 4, to obtain granular activated carbon for Example 5 (yield: 15.1%).

Example 6

Example 6 employed CELLULOSE BEADS D-200 (product of Daito Kasei Kogyo Co., Ltd.) as the spherical cellulose. To 250 g of the spherical cellulose there was added 500 mL of an ammonium phosphate water-soluble solution (5% concentration), and the mixture was allowed to stand for 12 hours. The water was then drained, and the mixture was dried at 120°C for 3 hours with a dryer. The total amount of the ammonium phosphate-treated spherical cellulose was placed in a cylindrical retort electric furnace, nitrogen was charged in, and then the temperature was raised to 600°C at 100°C/hr and held at that temperature for 1 hour for carbonization. The carbonized product was then heated to 900°C, water vapor was added and the mixture was held at that temperature for 30 minutes for activation (activation temperature: 900°C, activation time: 0.5 hour). The activated carbon was washed with dilute hydrochloric acid, nitrogen was charged in and heat treatment was performed in a cylindrical retort electric furnace at 750°C for 15 minutes to obtain granular activated carbon for Example 6 (yield: 27.1%).

Comparative Example 1

A 250 g portion of the spherical cellulose prepared in Example 1 was placed in a cylindrical retort electric furnace, nitrogen was charged in, and then the temperature was raised to 600°C at a rate of 100°C/hr and held at 600°C for 1 hour for carbonization. The carbonized product was then heated to 900°C, water vapor was added and the mixture was held at 900°C for 1 hour for activation (activation temperature: 900°C, activation time: 1 hour). The activated carbon was washed with dilute hydrochloric acid, nitrogen was charged in and heat treatment was performed in a cylindrical retort electric furnace at 600°C for 1 hour to obtain granular activated carbon for Comparative Example 1 (yield: 11.6%).

Comparative Example 2

After loading 250 g of a spherical phenol resin (AH-1131, product of Lignyte, Inc.) into a reaction cylindrical retort electric furnace, nitrogen was charged in to adjust the oxygen concentration to no greater than 3%, and then the temperature was raised to 600°C at 100°C/hr and that temperature was held for 1 hour for carbonization. The carbonized product was then heated to 900°C, water vapor was added and the mixture was held at that temperature for 1 hour for activation. The activated carbon was washed with dilute hydrochloric acid, and then nitrogen was charged in and heat treatment was performed in a cylindrical retort electric furnace at 600°C for 1 hour to obtain granular activated carbon for Comparative Example 2 (yield: 45.6%).

Comparative Example 3

Granular activated carbon for Comparative Example 3 was obtained by the same method as Comparative Example 2, except that the activation time of Comparative Example 2 was changed to 3 hours (yield: 29.3%).
The granular activated carbons of each of the examples and comparative examples are listed in Tables 1 to 3, together with the reaction conditions for the granular activated carbon. In order from the top of the tables are the carbonization conditions (°C x hr), activation conditions (°C x hr), temperature elevating conditions (°C/hr), yield (%), mean pore diameter (nm), BET specific surface area ($m^2$/g), mean particle size ($\mu$m), packing density (g/mL), mercury pore volume and $N_2$ pore volume (both mL/g), surface oxide content (meq/g), iodine adsorptive power (mg/g), and the creatinine, arginine and trypsin adsorption rates (%). The indication "<0.1" in the tables denotes a value below the measuring threshold value.

Table 1

| | Example 1 | Example 2 | Example 3 |
|---|---|---|---|
| Carbonizing conditions (°C x hr) | 600°C x 1 hr | 600°C x 1 hr | 600°C x 1 hr |
| Activating conditions (°C x hr ) | 900°C x 1 hr | 750°C x 2 hr | 750°C x 8 hr |
| Temperature increase conditions (°C/hr ) | 100°C/hr | 100°C/hr | 100°C/hr |
| Yield (%) | 19.6 | 30.1 | 22.6 |
| Mean pore diameter Dp (nm) | 1.94 | 1.83 | 1.92 |
| BET specific surface area Sa ($m^2$/g) | 1411 | 845 | 1139 |
| Mean particle size ($\mu$m) | 969 | 1002 | 920 |
| Packing density (g/mL) | 0.57 | 0.75 | 0.64 |
| Mercury pore volume $V_{me}$ (mL/g) | 0.11 | 0.07 | 0.09 |
| $N_2$ Pore volume $V_{ni}$ (mL/g) | 0.684 | 0.386 | 0.546 |
| Surface oxide content (meq/g) | 0.38 | 0.45 | 0.35 |
| Iodine adsorptive power (mg/g) | 1210 | 810 | 1040 |
| Creatinine adsorption (%) | 93.8 | 91.4 | 92.6 |
| Arginine adsorption (%) | 74.0 | 33.5 | 77.1 |
| Trypsin adsorption (%) | <0.1 | <0.1 | <0.1 |

Table 2

| | Example 4 | Example 5 | Example 6 |
|---|---|---|---|
| Carbonizing conditions (°C x hr ) | 600°C x 1 hr | 600°C x 1 hr | 600°C x 1 hr |
| Activating conditions (°C x hr ) | 900°C x 0.75 hr | 900°C x 0.75 hr | 900°C x 0.5 hr |
| Temperature increase conditions (°C/hr ) | 100°C/hr | 100°C/hr | 100°C/hr |
| Yield (%) | 14.4 | 15.1 | 27.1 |
| Mean pore diameter Dp (nm) | 2.11 | 2.04 | 1.82 |
| BET specific surface area Sa ($m^2$/g) | 1471 | 1386 | 895 |
| Mean particle size ($\mu$m) | 580 | 388 | 115 |
| Packing density (g/mL) | 0.45 | 0.50 | 0.77 |
| Mercury pore volume $V_{me}$ (mL/g) | 0.28 | 0.16 | 0.03 |
| $N_2$ Pore volume $V_{ni}$ (mL/g) | 0.776 | 0.706 | 0.408 |
| Surface oxide content (meq/g) | 0.19 | 0.48 | 0.14 |
| Iodine adsorptive power (mg/g) | 1190 | 1070 | 1050 |
| Creatinine adsorption (%) | 91.3 | 90.7 | 92.9 |
| Arginine adsorption (%) | 91.5 | 87.7 | 48.4 |
| Trypsin adsorption (%) | 5.1 | 3.1 | 1.7 |

Table 3

| | Comparative Example 1 | Comparative Example 2 | Comparative Example 3 |
|---|---|---|---|
| Carbonizing conditions (°C x hr ) | 600°C x 1 hr | 600°C x 1 hr | 600°C x 1 hr |
| Activating conditions (°C x hr ) | 900°C x 1 hr | 900°C x 1 hr | 900°C x 3 hr |
| Temperature increase conditions (°C/hr ) | 100°C/hr | 100°C/hr | 100°C/hr |
| Yield (%) | 11.6 | 45.6 | 29.3 |
| Mean pore diameter Dp (nm) | 3.64 | 1.76 | 1.83 |
| BET specific surface area Sa ($m^2$/g) | 1132 | 862 | 1210 |
| Mean particle size ($\mu$m) | 770 | 360 | 300 |
| Packing density (g/mL) | 0.42 | 0.72 | 0.62 |
| Mercury pore volume $V_{me}$ (mL/g) | 0.38 | 0.03 | 0.10 |
| $N_2$ Pore volume $V_{ni}$ (mL/g) | 1.030 | 0.380 | 0.554 |
| Surface oxide content (meq/g) | 0.61 | 0.03 | 0.10 |
| Iodine adsorptive power (mg/g) | 1010 | 910 | 1250 |
| Creatinine adsorption (%) | 93.8 | 90.4 | 93.4 |
| Arginine adsorption (%) | 83.5 | 42.2 | 78.5 |
| Trypsin adsorption (%) | 47.8 | <0.1 | 5.4 |

Results and discussion

The granular activated carbon of each of the examples exhibited physical properties generally equivalent to those of existing spherical phenol resin-derived granular activated carbon (Comparative Example 2). It was therefore demonstrated that granular cellulose is useful as a substitute starting material for spherical phenol resin. In addition, the granular activated carbon of each of the examples exhibited selective adsorption, having increased adsorption rates for toxic substances such as creatinine and arginine while minimizing adsorption of essential substances such as trypsin. In particular, Examples 1 to 3 adsorbed essentially no trypsin, while Examples 4 to 6 had extremely low adsorption of trypsin. The results of adsorption measurement indicated that the granular activated carbon of each of the examples have highly superior selective adsorption performance.

The packing densities of the granular activated carbons of the examples also suggested the possibility of a medical adsorbent in a highly compact dosage form, regardless of the type. It may therefore be considered to be suitable as a medical adsorbent for efficient absorption of toxic substances.

Industrial Applicability

Granular activated carbon exhibiting physical properties according to the invention reaches the digestive organs via oral administration, and is highly promising as a medical adsorbent that efficiently absorbs and eliminates toxic substances while limiting absorption of substances that are essential to the human body.

**Claims**

1.  A medical adsorbent that comprises granular activated carbon that is activated carbon obtained by carbonization and activation of refined cellulose or regenerated cellulose, and that has a mean pore diameter of 1.5 to 2.2 nm, a BET specific surface area of 700 to 3000 $m^2$/g, a mean particle size of 100 to 1100 $\mu$m, a surface oxide content of 0.05 meq/g or greater, and a packing density of 0.4 to 0.8 g/mL.

2.  The medical adsorbent according to claim 1 for use as a therapeutic or prophylactic agent for kidney disease or liver disease by oral administration.

3. A method for producing a medical adsorbent whereby in production of granular activated carbon according to claim 1, refined cellulose or regenerated cellulose is carbonized at 300°C to 700°C under a nitrogen atmosphere, and then subjected to steam-activation at 750°C to 1000°C, acid cleaning and heat treatment at 500°C to 800°C.

4. The method for producing a medical adsorbent according to claim 3, wherein the refined cellulose or regenerated cellulose is impregnated into ammonium phosphate or a metal phosphate, before being carbonized.

**Patentansprüche**

1. Medizinisches Adsorptionsmittel, das gekörnte Aktivkohle umfasst, die Aktivkohle ist, die durch Karbonisieren und Aktivieren von raffinierter Cellulose oder regenerierter Cellulose erhalten wird, und die einen mittleren Porendurchmesser von 1,5 bis 2,2 nm, eine BET-spezifische Oberfläche von 700 bis 3000 $m^2$/g, eine mittlere Teilchengröße von 100 bis 1100 $\mu$m, einen Oberflächenoxidgehalt von 0,05 meq/g oder größer und eine Packungsdichte von 0,4 bis 0,8 g/mL aufweist.

2. Medizinisches Adsorptionsmittel nach Anspruch 1 für die Verwendung als ein therapeutisches oder prophylaktisches Mittel für eine Nierenerkrankung oder Lebererkrankung durch orale Verabreichung.

3. Verfahren für die Herstellung eines medizinischen Adsorptionsmittels, wobei in der Herstellung der gekörnten Aktivkohle nach Anspruch 1, raffinierte Cellulose oder regenerierte Cellulose bei 300°C bis 700°C in einer Stickstoffatmosphäre karbonisiert wird und dann einer Dampfaktivierung bei 750°C bis 1000°C, einer Säurereinigung und einer Wärmebehandlung bei 500°C bis 800°C unterzogen wird.

4. Verfahren für die Herstellung eines medizinischen Adsorptionsmittels nach Anspruch 3, wobei die raffinierte Cellulose oder regenerierte Cellulose, bevor sie karbonisiert wird, in Ammoniumphosphat oder einem Metallphosphat imprägniert wird.

**Revendications**

1. Adsorbant à usage médical qui comprend du charbon actif granulaire qui est un charbon actif obtenu par carbonisation et activation de cellulose raffinée ou de cellulose régénérée, et qui a un diamètre de pore moyen de 1,5 à 2,2 nm, une aire spécifique par la méthode B.E.T. de 700 à 3000 $m^2$/g, une taille de particule moyenne de 100 à 1100 $\mu$m, une teneur en oxyde de surface de 0,05 méq/g ou plus, et une densité de tassement de 0,4 à 0,8 g/mL.

2. Adsorbant à usage médical selon la revendication 1 pouvant être utilisé à titre d'agent thérapeutique ou prophylactique pour traiter ou prévenir une maladie rénale ou une maladie hépatique par administration par voie orale.

3. Procédé de production d'un adsorbant à usage médical **caractérisé en ce que** lors de la production du charbon actif granulaire selon la revendication 1, la cellulose raffinée ou la cellulose régénérée est carbonisée à 300-700 °C dans une atmosphère d'azote, puis soumise à une activation par la vapeur à 750-1000 °C, à un nettoyage acide et à un traitement thermique à 500-800 °C.

4. Procédé de production d'un adsorbant à usage médical selon la revendication 3, dans lequel la cellulose raffinée ou la cellulose régénérée est imprégnée dans du phosphate d'ammonium ou un phosphate métallique, avant d'être carbonisée.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1440692 B1
- JP 3835698 B
- JP 2008 A
- JP 303193 A
- JP 2008303193 A
- JP 6135841 A
- JP 2002 A
- JP 308785 A
- JP 2002308785 A